# EUROPEAN PATENT APPLICATION

(11) **EP 2 446 829 A2**
(43) Date of publication of application: **02.05.2012**
(21) Application number: 11250760.3
(22) Date of filing: 09.09.2011
(51) Int. Cl.: A61B 17/00

(54) **Applicator tips having mixing ball**

(30) Priority: 28.10.2010 US 914099
(71) Applicant: Tyco Healthcare Group LP, New Haven, CT 06511 (US)
(72) Inventor: Sargeant, Timothy, Guilford, CT 06437 (US); Desai, Arpan, Hamden, CT 06514 (US); Stopek, Joshua, Guilford, CT 06437 (US)
(74) Representative: Soames, Candida Jane

(57) **Abstract**

Applicator tips in accordance with the present disclosure include a body portion and dispensing tip. The body portion includes a proximal end having an inlet adapted for fluid communication with a fluid source, a distal end including an opening, and a mixing chamber disposed therebetween. The mixing chamber includes at least one mixing ball and at least one biologically active agent disposed therein. The dispensing tip defines a lumen therethrough. The lumen is in fluid communication with the opening of the distal end of the body portion and includes an outlet for passage of a fluid therethrough.

## Description

### BACKGROUND

The present disclosure relates to applicator tips for use with fluid applicator devices, and in particular to applicator tips containing a drug or other biologically active agent which is dispensed with the fluid being delivered therethrough.

Fluid applicators, e.g., syringes, are commonly used for injecting fluid mixtures such as drugs, nutrients, and the like, into the body. The fluid mixture is often prepared by dissolving or mixing a dry component, e.g., drugs, nutrients, etc., into a carrier solution or fluid component, e.g., saline solution. The fluid mixture is then loaded into the fluid applicator and discharged through an applicator tip.

While some mixtures may be prepared ahead of time, it is often advantageous, if not necessary, to mix the components immediately prior to use. Thus, in order to apply the mixture, the additional steps of preparing the mixture and loading the fluid applicator with the prepared mixture may need to be taken, or a pre-packaged fluid applicator containing the separate mixture components may be utilized.

It would be advantageous to have the ability to tailor the selection of biologically active agents for incorporation into a fluid mixture. This would allow for individualized treatment of the patient by catering to the patient's specific needs.

### SUMMARY

The present disclosure provides applicator tips for use with fluid applicator devices. In embodiments, an applicator tip of the present disclosure may include a body portion including a proximal end having an inlet adapted for fluid communication with a fluid source, a distal end including an opening, and a mixing chamber disposed therebetween, the mixing chamber possessing at least one mixing ball and at least one biologically active agent disposed therein; and a dispensing tip defining a lumen therethrough, the lumen being in fluid communication with the opening of the distal end of the body portion and including an outlet for passage of a fluid therethrough. In embodiments, the biologically active agent may be coated on at least a portion of the mixing ball.

Methods for delivering fluids and biologically active agents are also provided. In embodiments, a method of the present disclosure may include introducing to a target tissue site a delivery device including a fluid applicator including at least one fluid chamber containing a fluid and a fluid actuator for dispensing the fluid from the fluid chamber; and an applicator tip including a mixing chamber fluidly coupled to at least one fluid chamber of the fluid applicator, the mixing chamber containing at least one mixing ball and at least one biologically active agent and being fluidly coupled to a dispensing tip. The fluid actuator of the fluid applicator may be activated to urge fluid from the at least one fluid chamber of the fluid applicator into the mixing chamber of the applicator tip, such that movement of the fluid causes the mixing ball to move within the mixing chamber and mix the fluid with the biologically active agent disposed therein. The fluid and biologically active agent may then be dispensed through the dispensing tip to the target tissue site.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1A is a front view of an applicator tip in accordance with an embodiment of the present disclosure;

FIG. 1B is a cross-sectional view of the applicator tip of FIG. 1A;

FIG. 2 is a cross-sectional view of an applicator tip including a static mixer in accordance with another embodiment of the present disclosure;

FIG. 3 is a cross-sectional view of an applicator tip including a biological active agent freely disposed in a mixing chamber of the applicator tip in accordance with one embodiment of the present disclosure;

FIG. 4A is a cross-sectional view of an applicator tip including a biologically active agent coated on a mixing ball in a mixing chamber of the applicator tip in accordance with another embodiment of the present disclosure;

FIG. 4B is a schematic illustration of an embodiment of coating the biologically active agent on the mixing ball of FIG. 4A;

FIG. 4C is a schematic illustration of another embodiment of coating the biologically active agent on the mixing ball of FIG. 4A;

FIG. 5A is a cross-sectional view of a mixing ball coated with a biologically active agent in accordance with an embodiment of the present disclosure;

FIG. 5B is a schematic illustration of a mold for coating the mixing ball of FIG. 5A;

FIG. 6 is a cross-sectional view of a mixing ball coated with a biologically active agent in accordance with another embodiment of the present disclosure;

FIG. 7 is a cross-sectional view of a mixing ball coated with a biologically active agent in accordance with yet another embodiment of the present disclosure;

FIG. 8 is a perspective view of a fluid applicator for use with an applicator tip in accordance with an embodiment of the present disclosure;

FIG. 9 is a perspective view of a fluid applicator for use with an applicator tip in accordance with another embodiment of the present disclosure;

FIG. 10A is a perspective view of a fluid applicator for use with an applicator tip in accordance with yet another embodiment of the present disclosure;

FIG. 10B is a cross-sectional view of the fluid applicator of FIG. 10A;

FIG. 11A is a perspective view of a fluid applicator for use with an applicator tip in accordance with another embodiment of the present disclosure;

FIG. 11B is a partial cross-sectional view of the fluid applicator of FIG. 11A; and

FIG. 12 is a perspective view of a fluid applicator for use with an applicator tip in accordance with yet another embodiment of the present disclosure.

### DETAILED DESCRIPTION

Applicator tips in accordance with the present disclosure include a mixing chamber and a dispensing tip. The mixing chamber contains a biologically active agent configured for attachment with a fluid applicator device. Fluid may be delivered into the mixing chamber of the applicator tip from the fluid applicator device, whereby the movement and flow of the fluid, as well as a mixing ball positioned within the mixing chamber, causes the biologically active agent to mix therewith. The fluid and biologically active agent may then be delivered through the dispensing tip to a target site.

The fluid contained within the fluid applicator devices may be any flowable material with medical utility. In embodiments, the fluid may be a salt solution, including a saline solution, precursors to a hydrogel, a sealant, an adhesive, or any other solution, semi-solid, or paste. Thus, the fluid delivered through the applicator tip may be utilized as adhesives, sealants, coatings for implants, drug delivery devices, wound fillers, and/or tissue augmentation materials, among other uses as envisioned by those skilled in the art.

Embodiments of the presently disclosed applicator tips will now be described in detail with reference to the drawing figures wherein like reference numerals identify similar or identical structural elements. As shown in the drawings and described throughout the following description, and as is traditional, the term "proximal" refers to the end of the apparatus which is closer to the user and the term "distal" refers to the end of the apparatus which is further away from the user.

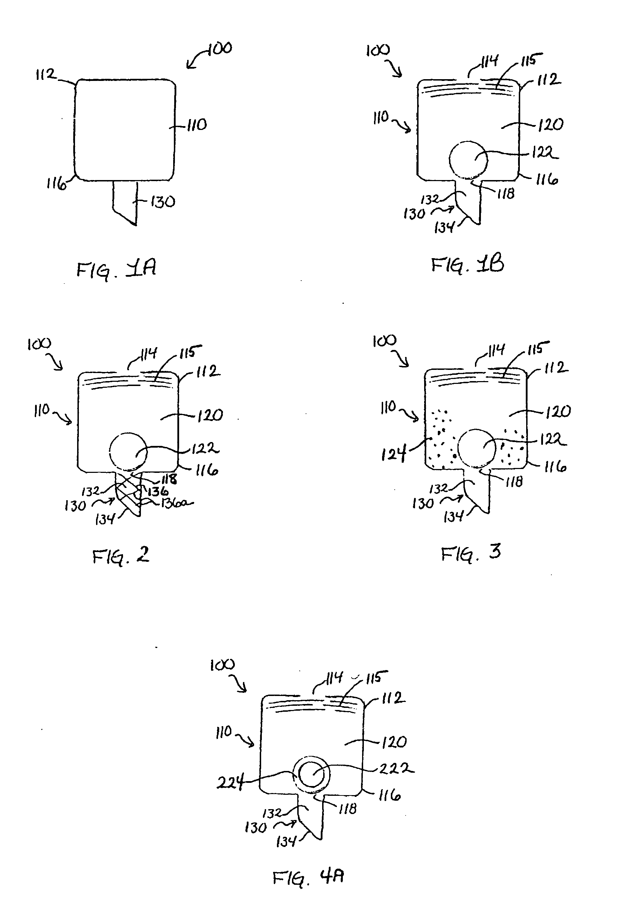

Referring initially to FIGS. 1 A and 1B, an applicator tip in accordance with the present disclosure is shown generally as reference numeral 100. Applicator tip 100 includes a body portion 110 and a dispensing tip 130. Body portion 110 includes a proximal end 112, a distal end 116, and a mixing chamber 120 defined therebetween.

The proximal end 112 of body portion 110 is configured and dimensioned for releasably securing applicator tip 100 to a fluid source, such as a fluid applicator device, as will be described in further detail below. An inlet 114 is provided at the proximal end 112 for fluid communication between the mixing chamber 120 and the fluid applicator device. As illustrated in FIG. 1B, the inner surface of the proximal end 112 includes threads 115 for establishing a screw-fit connection with an outer surface of a fluid applicator device to attain a fluid tight seal between the two components. It is envisioned that other conventional mechanical means may be utilized to releasably secure an applicator tip with a fluid applicator device including, for example, bayonet couplings, snap fit arrangements, friction fittings, tongue and groove configurations, cam-lock mechanisms, and other male/female fasteners within the purview of those skilled in the art. Alternatively, proximal end 112 of applicator tip 100 may have no mating member but may be chemically couplable to a fluid applicator device via use of adhesives or permanently couplable via ultrasonic welding, and the like. In other embodiments, an external fastener may be positioned between, or over, the applicator tip and the fluid applicator device for forming a fluid tight seal between the two components.

Distal end 116 includes opening 118 for fluidly connecting mixing chamber 120 of body portion 110 with dispensing tip 130. Mixing chamber 120 includes at least one mixing ball 122 and at least one biologically active agent (not shown). While the mixing ball 122 is illustrated as being spherical in shape, it is envisioned that the mixing ball 122 may be any shape within the purview of those skilled in the art, such as ovoidal, ellipsoidal, and/or amorphous, for example. The diameter of mixing ball 122 is larger than the diameter of the inlet 114 and the opening 118 of mixing chamber 120, such that mixing ball 122 is confined within the mixing chamber 120 and is free to move and/or rotate therein.

The mixing ball 122 may be fabricated from a variety of materials including polymers, plastics, metals, and ceramics. Suitable polymers which the mixing ball may be fabricated of include, but are not limited to, polyolefins such as polyethylene (including ultra high molecular weight polyethylene) and polypropylene (including atactic, isotactic, syndiotactic, and blends thereof); polyethylene glycols (PEGs); polyethylene oxides; copolymers of polyethylene and polypropylene; polyisobutylene and ethylene-alpha olefin copolymers; fluorinated polyolefins such as fluoroethylenes, fluoropropylenes, fluoroPEGs, and polytetrafluoroethylene; polyamides; polyamines; polyimines; polyesters such as polyethylene terephthalate, polyethylene naphthalate, polytrimethylene terephthalate, and polybutylene terephthalate; polyethers; polybutesters; polytetramethylene ether glycol; 1,4-butanediol; polyurethanes; acrylic polymers; methacrylics; vinyl halide polymers and copolymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins such as ethylene-methyl methacrylate copolymers; acrylonitrile-styrene copolymers; acrylonitrile butadiene styrene resins; ethylene-vinyl acetate copolymers; alkyd resins; polycarbonates; polyoxymethylenes; polyphosphazines; polyimides; epoxy resins; aramids; silicones; and copolymers and combinations thereof.

Additionally, the mixing ball 122 may be fabricated of biodegradable polymers. For example, the mixing ball 122 may be fabricated of polymers, copolymers and oligomers of glycolide, dl-lactide, 1-lactide, caprolactone, dioxanone, trimethylene carbonate, poly(hydroxy acid)s, poly(orthocarbonate)s, poly(anhydride)s, poly(lactone)s, poly(aminoacid)s, poly(carbonate)s, poly(phosphonate)s, combinations thereof, and the like.

Returning to FIGS. 1A and 1B, dispensing tip 130 defines a lumen 132 therethrough and includes outlet 134 for discharging fluid toward a target site. The dispensing tip 130 may range in size, length, and shape, depending on the desired pressure, flow rate, and spray pattern of the liquid to be dispensed. Factors including the viscosity of the fluid and the diameter of the tip may also affect the spraying characteristics of the dispensing tip 130.

The dispensing tip 130 may include a static mixer 136 for mixing the fluid(s) and biologically active agent(s) as illustrated in FIG. 2. The static mixer 136 may include one or more baffles 136a or the like. The baffles 136a may be fixed and remain substantially motionless as the fluid and biologically active agent pass therethrough. In embodiments, as the fluid and biologically active agent are pushed into the dispensing tip 130 and through the static mixer 136, the flow of the fluid is interrupted and diverted upon each encounter with a baffle 136a thereby causing turbulent flow in the mixing chamber 120 and blending of the fluid with the biologically active agent.

The biologically active agent disposed within the mixing chamber may be any substance or mixture of substances that have clinical use. The biologically active agent may invoke a biological action, exert a biological effect, or play a role in one or more biological processes. Alternatively, the biologically active agent may not have pharmacological activity per se, e.g., a dye. It is envisioned that any biologically active agent may be utilized with the applicator tips of the present disclosure. The type and amount of biologically active agent used will depend, among other factors, on the particular site and condition to be treated and the type of fluid being dispensed from the fluid applicator device.

Examples of classes of biologically active agents which may be utilized in accordance with the present disclosure include anti-adhesives, antimicrobials, analgesics, antipyretics, anesthetics, antiepileptics, antihistamines, anti-inflammatories, cardiovascular drugs, diagnostic agents, sympathomimetics, cholinomimetics, antimuscarinics, antispasmodics, hormones, muscle relaxants, adrenergic neuron blockers, antineoplastics, immunogenic agents, immunosuppressants, gastrointestinal drugs, diuretics, steroids, lipids, lipopolysaccharides, polysaccharides, platelet activating drugs, clotting factors, and enzymes.

Suitable antimicrobial agents which may be included as a biologically active agent include triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil; nonoxynol 9; fusidic acid; cephalosporins; and combinations thereof. In addition, antimicrobial proteins and peptides such as bovine lactoferrin and lactoferricin B may be included as a biologically active agent in the present disclosure.

Other biologically active agents include: local anesthetics; non-steroidal antifertility agents; parasympathomimetic agents; psychotherapeutic agents; tranquilizers; decongestants; sedative hypnotics; steroids; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists such as naltrexone and naloxone; anti-cancer agents; anti-convulsants; anti-emetics; antihistamines; anti-inflammatory agents such as hormonal agents, hydrocortisone, prednisolone, prednisone, non-hormonal agents, allopurinol, indomethacin, phenylbutazone and the like; prostaglandins and cytotoxic drugs; chemotherapeutics; estrogens; antibacterials; antibiotics; anti-fungals; anti-virals; anticoagulants; anticonvulsants; antidepressants; antihistamines; and immunological agents.

Other examples of suitable biologically active agents include viruses and cells; peptides; polypeptides and proteins, as well as analogs, muteins, and active fragments thereof; immunoglobulins; antibodies; cytokines (e.g., lymphokines, monokines, chemokines); blood clotting factors; hemopoietic factors; interleukins (IL-2, IL-3, IL-4, IL-6); interferons (β-IFN, α-IFN and γ-IFN); erythropoietin; nucleases; tumor necrosis factor; colony stimulating factors (e.g., GCSF, GM-CSF, MCSF); insulin; antitumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); protein inhibitors; protein antagonists; protein agonists; nucleic acids such as antisense molecules, DNA, and RNA; oligonucleotides; polynucleotides; ribozymes; naturally occurring polymers including proteins such as collagen and derivatives of various naturally occurring polysaccharides such as glycosaminoglycans; peptide hydrolases such as elastase, cathepsin G, cathepsin E, cathepsin B, cathepsin H, cathepsin L, trypsin, pepsin, chymotrypsin, γ-glutamyltransferase (γ -GTP) and the like; sugar chain hydrolases such as phosphorylase, neuraminidase, dextranase, amylase, lysozyme, oligosaccharase and the like; oligonucleotide hydrolases such as alkaline phosphatase, endoribonuclease, endodeoxyribonuclease and the like.

In embodiments, the biologically active agent is a biologic or cell specific ligand capable of attracting or recruiting specific cell types, such as smooth muscle cells, stem cells, immune cells, and the like.

In embodiments, the biologically active agent may be a growth factor, such as transforming growth factors (TGFs), fibroblast growth factors (FGFs), platelet derived growth factors (PDGFs), epidermal growth factors (EGFs), connective tissue activated peptides (CTAPs), osteogenic factors, and biologically active analogs, fragments, and derivatives of such growth factors. In embodiments, members of the transforming growth factor (TGF) supergene family, which are multifunctional regulatory proteins, are utilized. Members of the TGF supergene family include the beta transforming growth factors (for example, TGF-β1, TGF-β2, TGF-β3); bone morphogenetic proteins (for example, BMP-1, BMP-2, BMP-3MP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (IGF)); Inhibins (for example, Inhibin A, Inhibin B); growth differentiating factors (for example, GDF-1); and Activins (for example, Activin A, Activin B, Activin AB).

In embodiments, the biologically active agent may be an imaging agent such as iodine or barium sulfate, or fluorine, to allow visualization of the fluid at the time of application or thereafter through the use of imaging equipment, including X-ray, MRI, and CAT scan equipment. Other imaging agents which may be included are within the purview of those skilled in the art and include, but are not limited to, substances suitable for use in medical implantable medical devices, such as FD&C dyes 3 and 6, eosin, methylene blue, indocyanine green, or colored dyes normally found in synthetic surgical sutures. Suitable colors include green and/or blue because such colors may have better visibility in the presence of blood or on a pink or white tissue background. In embodiments, the biologically active agent may include a dye to allow visualization of the fluid in the mixing chamber to ensure that the biologically active agent is mixing with the fluid prior to dispensing the fluid through the dispensing tip.

The biologically active agent may be disposed within the mixing chamber in a variety of ways. As illustrated in FIG. 3, a biologically active agent 124 may be freely disposed in a mixing chamber 122 in any form, such as powder or liquid, such that when fluid is introduced into the mixing chamber from the inlet 114 in the proximal end 112 of the body portion 110 the fluid carries the biologically active agent 124 out through the dispensing tip 130. In other embodiments, a biologically active agent may be disposed on a surface of the interior of the mixing chamber such that when fluid contacts the surface, the agent is mixed within the fluid. Alternatively, the biologically active agent may be disposed on, or in, the mixing ball, or the biologically active agent may form the mixing ball itself. While the mixing ball is illustrated in embodiments of the present disclosure as being completely coated by the biologically active agent, it is envisioned that only a portion or portions of the mixing ball may be coated. Moreover, in addition to being coated with a composition containing one or more biologically active agents, portions of the mixing ball may be coated with different biologically active agents (or mixtures of biologically active agents). For example, one half of the mixing ball may be coated with a first biologically active agent, and the other half of the mixing ball may be coated with a second biologically active agent. Similarly, a mixing ball may have an inner coating with one biologically active agent and an outer coating with a second biologically active agent. In such an embodiment with coextensive layered coatings, the second biologically active agent will be released first, upon dispensing a fluid, with the first biologically active agent being released after the second biologically active agent has been released.

Referring now to FIG. 4A, mixing ball 222 may be coated with biologically active agent 224. In embodiments, the mixing ball is substantially solid and includes a continuous outer surface. In other embodiments, the outer surface of the mixing ball may be modified prior to application of the biologically active agent thereto to aid in the adhesion of the biologically active agent to the mixing ball. For example, in embodiments, the surface of the mixing ball may be roughened or plasma treated.

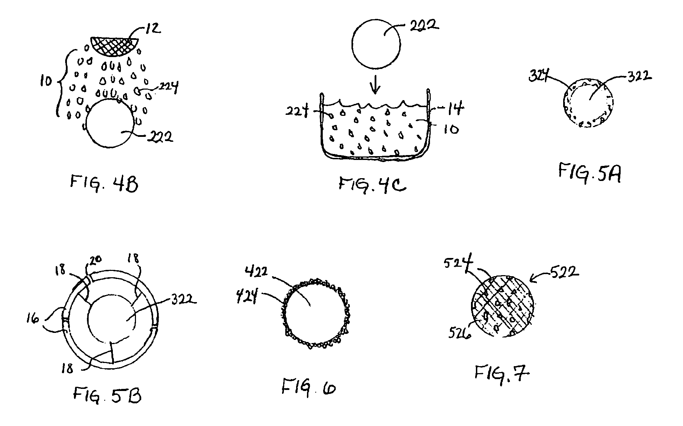

The biologically active agent 224 may be applied to the mixing ball 222 utilizing techniques within the purview of those skilled in the art, e.g., dipping, wiping, spraying, total immersion, solvent evaporation, etc. As illustrated in FIG. 4B, an aqueous solvent or solution 10 containing the biologically active agent 224 may be sprayed onto the mixing ball 222 via dispenser or sprayer 12. After spraying and coating, the solution 10 containing the biologically active agent 224 onto the surface of the mixing ball 222, the solution may be dried or allowed to cure. In embodiments, the drying temperatures may be from about 1°C to about 70°C.

In other embodiments, as illustrated in FIG. 4C, the mixing ball 222 may be submerged into a vessel 14 containing a solution 10 containing the biologically active agent 224 by moving the mixing ball 222 in the direction of the arrow for a sufficient amount of time to coat the surface of the mixing ball 222. Thereafter, the mixing ball 222 is removed from the vessel 14 and is dried or cured to form a mixing ball 222 that is coated with the biologically active agent 224 as shown in FIG. 4A.

In yet another embodiment, a biologically active agent may be contained, or entrapped, in a thin layer of a hydrophilic or degradable polymer. The layer containing the biologically active agent may be applied to a mixing ball and cured.

A biologically active agent 324 may also be lyophilized onto the surface of a mixing ball 322 to form a porous coating as illustrated in FIG. 5A. In embodiments, a viscous solution containing the biologically active agent 324 may be applied to the mixing ball 322, for example by spraying or dip coating as described above, and the biologically active agent 324 may then be freeze-dried to form a coating on the mixing ball 322. Suitable techniques for lyophilization or freeze-drying compositions are within the purview of those skilled in the art.

Other methods for applying a bioactive agent to a mixing ball include the use of a mold. As illustrated in FIG. 5B, mixing ball 324 may be positioned in a mold 16. Positioning elements 18, such as prongs, may hold the mixing ball 322 substantially in the center of mold 16. A solution containing the biologically active agent (not shown) may be introduced through an opening 20 in the mold 16 to surround the mixing ball 322 positioned therein. The solution is lyophilized to form a porous coating as illustrated in FIG. 5A, and the mold 16 is removed. It is envisioned that the size, shapes, and dimensions of the mold 16 may vary, thereby creating a porous coating of the biologically active agent 324 of any suitable shape or size on the mixing ball 322.

A powder coating of a biologically active agent 424 may also be utilized to coat a mixing ball 422 as illustrated in FIG. 6. A biologically active agent 424 in a dry powdered form may be applied to a positively or negatively charged mixing ball 422. Alternatively, the dry biologically active agent 424 may be electrostatically applied to a neutral mixing ball 422, for example, through spraying with an electrostatic gun or the like. Methods of applying a static coating are within the purview of those skilled in the art.

In embodiments, the biologically active agent may be loaded into liposomes or microspheres prior to coating a mixing ball via any of the techniques described above. In embodiments, the liposomes or microspheres may be functionalized to aid in coating the mixing ball. For example, a cationic lipid may be ionically bound to an anionic mixing ball, or an anionic lipid may be attracted to a cationic mixing ball. Polymers suitable for use in forming such liposomes or microspheres are within the purview of those skilled in the art.

Examples of negatively charged polymers that can be utilized in forming liposomes and/or microspheres include collagens, such as succinylated collagen, and glycosaminoglycan derivatives, such as sodium hyaluronate, keratan sulfate, keratosulfate, sodium chondroitin sulfate A, sodium dermatan sulfate B, sodium chondroitin sulfate C, heparin, esterified chondroitin sulfate C, and esterified heparin. Examples of positively charged polymers include collagens, such as methylated collagen, and glycosaminoglycan derivatives, such as esterified deacetylated hyaluronic acid, esterified deacetylated desulfated chondroitin sulfate A, esterified deacetylated desulfated chondroitin sulfate C, deacetylated desulfated keratin sulfate, deacetylated desulfated keratosulfate, esterified desulfated heparin, and chitosan.

In embodiments, lipids or surfactants (e.g., nonionic surfactants, mixtures of anionic and cationic surfactants, polymers, or colloidal particles) may be provided, such as in powder form, to which an aqueous solution containing a biologically active agent may be added. The lipids and the aqueous solution containing the biologically active agent are then mixed. The lipids may self-assemble in the aqueous solution to form micelles incorporating the biologically active agent therein. The amount of biologically active agent incorporated into the lipid vesicles may vary, depending on the ratio of lipid to biologically active agent. The resulting composition may be purified, such as by ultracentrifugation, to separate the liposomes from the unencapsulated, or free, biologically active agent.

In other embodiments, microspheres encapsulating a biologically active agent may be formed by internally controlled gelation of an emulsion. An aqueous phase containing the biologically active agent is gradually mixed with an oil phase to form an emulsion. After sufficient time for solidification, the emulsion is broken and the microspheres containing the biologically active agent are collected.

As illustrated in FIG. 7, the mixing ball 522 may be porous such that the biologically active agent 524 may be immobilized both on the surface and within pores 526 of the mixing ball 522. For example, the biologically active agent 524 may be applied to the mixing ball 522 via any of the techniques provided above. The biologically active agent 524 may soak into the pores 526 of the mixing ball 522 or, through mechanical interactions such as wicking or capillary action, may pass into the pores 526 of the mixing ball 522. The biologically active agent 524 may then be dried or cured.

Alternatively, the mixing ball itself may be formed of the biologically active agent, such as by lyophilization or other molding or casting techniques. For example, a biodegradable polymer may be mixed with a biologically active agent and shaped to form a mixing ball, such as by lyophilization.

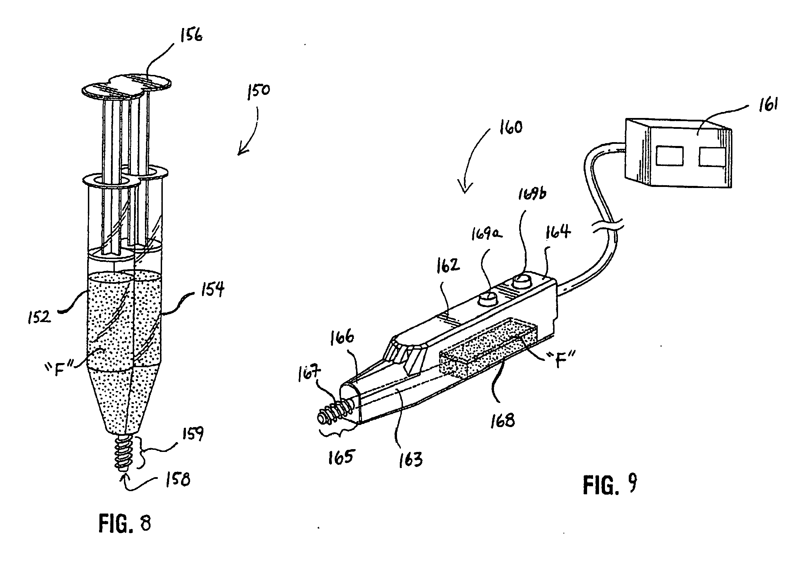

Fluid may be supplied to the mixing chamber of the applicator tip via a syringe or other fluid applicator device adapted for use with an applicator tip of the present disclosure. Examples of suitable fluid applicator devices which may be utilized with the applicator tip of the present disclosure include, but are not limited to, those depicted in the FIGS. 8-12.

For example, as shown in FIG. 8, in conjunction with FIGS. 1A and 1B, a syringe 150 is adapted for use with applicator tip 100. Syringe 150 includes two fluid chambers or barrels 152 and 154 for retaining fluids "F" therein. In embodiments, barrels 152 and 154 are provided to contain first and second components of a two-part mixture. Although illustrated as a dual barrel syringe, it is contemplated that syringe 150 may be a single barrel syringe or include more than two fluid chambers. Syringe 150 further includes a manually-operable plunger 156 for urging fluid(s) from barrels 152 and 154 and through open distal end 158 of syringe 150. In embodiments, the plunger 156 may be configured for independent operation with respect to each barrel. Distal end 158 of syringe 150 includes threads 159 for releasably coupling distal end 158 of syringe 150 to threads 115 in the proximal end 112 of applicator tip 100. As described above, it is also envisioned that syringe 150 may be releasably secured to the applicator tip 100 via any mating mechanism that provides a fluid tight seal between the syringe 150 and the applicator tip 100.

As can be appreciated, when applicator tip 100 is engaged with syringe 150, open distal end 158 of syringe 150 is in fluid communication with mixing chamber 120 of the body portion 110 of the applicator tip 100, such that, upon depression of plunger 156, fluid "F" is urged from barrels 152 and 154 of syringe 150 into mixing chamber 120, through dispensing tip 130, and out outlet 134 for delivery to a target site.

FIG. 9 illustrates another embodiment of a fluid applicator device for use with applicator tip 100. Fluid applicator device 160 generally includes a handle or housing 162 having a proximal end 164, a distal end 166, and at least one chamber 168 therein for retaining a fluid (or fluids) "F". Housing 162 is coupled to an energy source 161 for supplying electrical power to fluid applicator device 160 for dispensing fluid "F." Alternatively, fluid applicator device 160 may be battery-powered. A conduit 163 disposed within housing 162 is coupled to cavity 168, extends distally through housing 162 and ultimately couples to connector 165 disposed at distal end 166 of housing 162. Connector 165 includes threads 167 for releasably coupling distal end 166 of housing 162 to proximal end 112 of applicator tip 100 as described above. Housing 162 may also include a pair of buttons or controls 169a and 169b for controlling the operation of fluid applicator device 160. Controls 169a and 169b may be configured to, for example, activate/deactivate the delivery of fluid from cavity 168 through connector 165 and/or for switching between various modes of operation, e.g., high-power and low-power fluid dispensing modes.

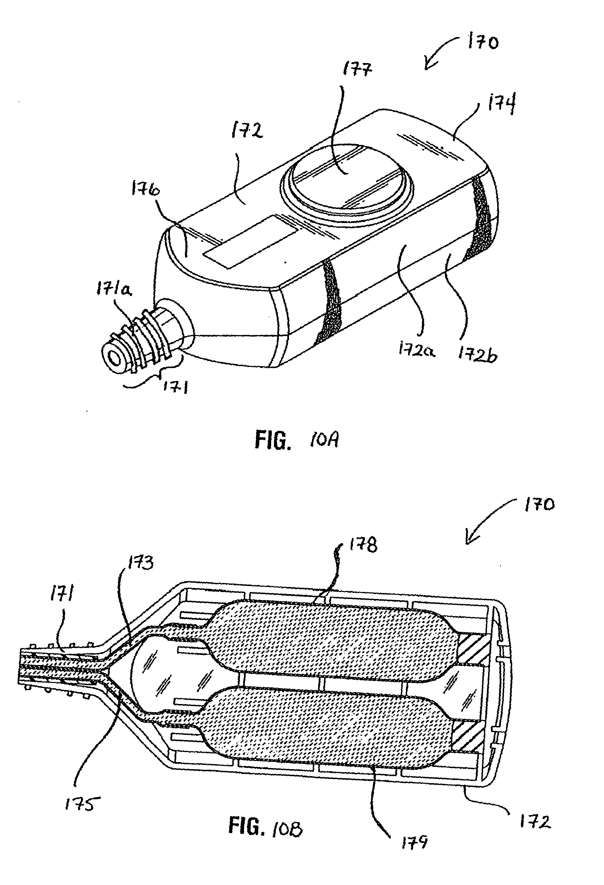

With reference now to FIGS. 10A and 10B, another embodiment of a fluid applicator device 170 is shown. Fluid applicator device 170 includes a housing 172 having a proximal end 174 and a distal end 176. Housing 172 is formed from two housing sections 172a and 172b which cooperate to form housing 172. Housing sections 172a and 172b are releasably engageable, e.g., via a snap-fit engagement, for providing access to the interior of housing 172.

As best seen in Fig. 10B, a pair of fluid chambers or reservoirs 178 and 179 are disposed within housing 172. Upon disengagement of housing sections 172a and 172b, fluid reservoirs 178 and 179 may be replaced and/or interchanged with new reservoirs containing the desired fluid. Alternatively, fluid reservoirs 178 and 179 may be refillable.

Housing 172 further includes a connector 171 disposed at distal end 174 thereof that is configured for engaging proximal end 112 of body portion 110 of applicator tip 100. Connector 171 may have threads 171a for releasably coupling connecter 171 to threads 115 in the proximal end 112 of applicator tip 100. A pair of conduits 173 and 175 disposed within housing 172 couples fluid reservoirs 178 and 179 respectively, with opening 114 of mixing chamber 120 of applicator tip 100 such that, upon actuation, fluid from fluid reservoirs 178 and 179 may be urged into mixing chamber 120 and through dispensing tip 130 for delivery to the desired site. Accordingly, housing 172 includes a selectively depressible actuator 177 for urging fluid from fluid reservoirs 178 and 179 through conduits 173 and 175 and into mixing chamber 120 of applicator tip 100.

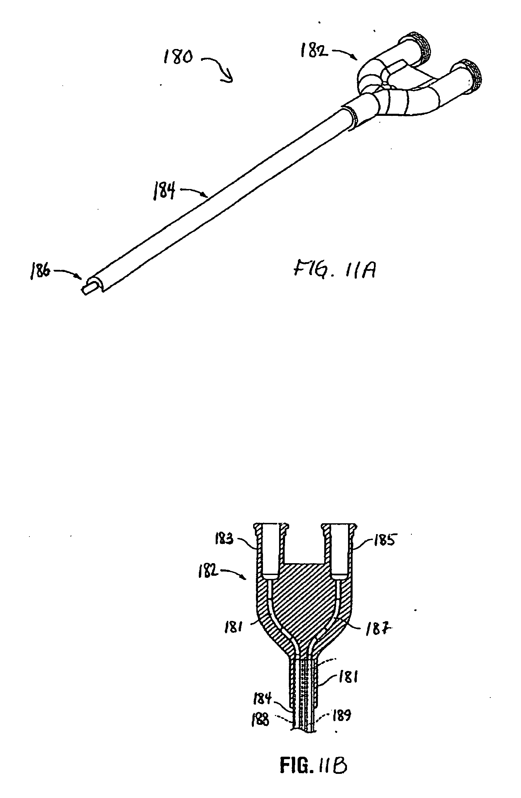

Referring now to FIGS. 11A and 11B, fluid applicator device 180 may include a manifold or base 182, an elongated shaft 184 extending from base 182, and a connector 186. Fluid chambers 183 and 185 are disposed within base 182 and are fluidly connected to conduits 181 and 187, respectively. In embodiments, fluid chambers 183 and 185 may be connected to other external sources of fluids (not shown) for administration to a patient. Elongated shaft 184 includes first and second lumens 188 and 189 extending the length thereof. Elongated shaft 184 is secured to distal extension 181 of manifold 182 such that first and second lumens 188 and 189 align with first and second conduits 181 and 187, respectively, to allow fluid flow therethrough. Alternatively, elongated shaft 184 may be integrally formed with manifold 182. Connector 186 may further include threads, grooves, detents, or other coupling means for secure engagement with proximal end 112 of applicator tip 100.

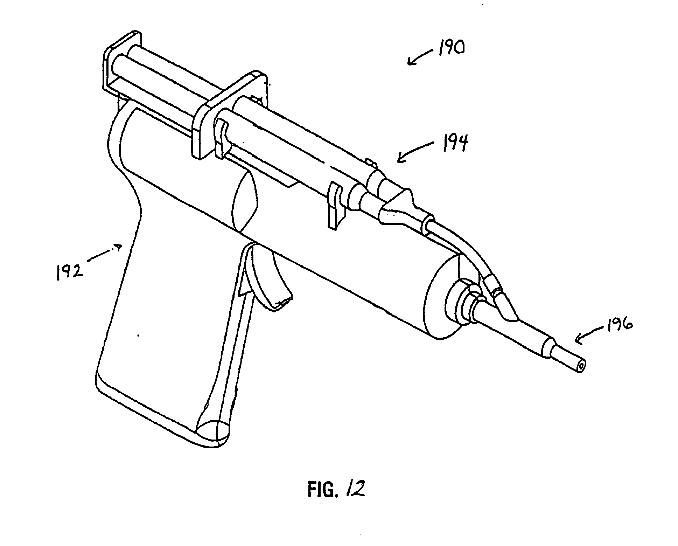

FIG. 12 illustrates a fluid applicator device 190 including a housing 192 configured for pistol-grip engagement by a user, a fluid supply source 194 operably connected to housing 192, and a connector 196 extending distally from housing 192. Fluid applicator device 190 is configured to urge fluid from the fluid supply source 194 through connector 196. Connector 196 may further include threads, grooves, detents, or other coupling means for secure engagement with proximal end 112 of applicator tip 100.

The operation of a fluid applicator device with an applicator tip of the present disclosure ensures the delivery of a fluid with a biologically active agent. In embodiments, the desired fluid applicator device is selected containing the desired fluid, depending upon the intended use. The applicator tip, including the desired amount and types of biologically active agents, as well as the desired number and types of mixing balls, is attached to the fluid applicator device. The delivery device may then be introduced into the target site of application. Upon activation of a fluid actuator (e.g., plungers, control buttons, and/or triggers), fluid is urged from the fluid containing chamber(s) of the fluid applicator device into the mixing chamber of the applicator tip containing the biologically active agent(s) and mixing ball(s). As noted above, the biologically active agent(s) may be in the mixing chamber itself, on or part of the material(s) used to fabricate the mixing ball, combinations thereof, and the like. The force of the fluid flow may cause the mixing ball(s) to move within the mixing chamber, thereby mixing the fluid with the biologically active agent. In embodiments in which two or more fluids are introduced into the mixing chamber, the movement of the fluid, as well as the movement of the mixing ball(s), causes mixing of the fluids with each other.

The fluid acts as a carrier for the biologically active agent(s). In embodiments, the introduction of fluid(s) within the mixing chamber causes the biologically active agent(s) to solubilize and diffuse into the fluid. The fluid(s) and biologically active agent(s) may then be urged into the dispensing tip and into the target site of application. In embodiments, a static mixer disposed within the dispensing tip may help ensure the homogeneous mixing of the fluid(s) and biologically active agent(s).

In embodiments, applicator tips of the present disclosure may further incorporate light, UV, visualization means (e.g., a camera), combinations thereof, and the like, in the applicator. In some embodiments, light may be used for activating a biologically active agent.

Persons skilled in the art will understand that the devices and methods specifically described herein and illustrated in the accompanying figures are non-limiting exemplary embodiments, and that the description, disclosure, and figures should be construed merely exemplary of particular embodiments. It is to be understood, therefore, that the present disclosure is not limited to the precise embodiments described, and that various other changes and modifications may be effected by one skilled in the art without departing from the scope or spirit of the disclosure. Additionally, it is envisioned that the elements and features illustrated or described in connection with one exemplary embodiment may be combined with the elements and features of another exemplary embodiment, without departing from the scope of the present disclosure, and that such modifications and variations are also intended to be included within the scope of the present disclosure. Accordingly, the subject matter of the present disclosure is not to be limited by what has been particularly shown and described, as modifications and variations are intended to come within the scope and spirit of the claims appended hereto.

The invention may be described by reference to the following numbered paragraphs:-
1. An applicator tip comprising:
   a body portion including a proximal end having an inlet adapted for fluid communication with a fluid source, a distal end including an opening, and a mixing chamber disposed therebetween, the mixing chamber possessing at least one mixing ball and at least one biologically active agent disposed therein; and
   a dispensing tip defining a lumen therethrough, the lumen being in fluid communication with the opening of the distal end of the body portion and including an outlet for passage of a fluid therethrough.
2. The applicator tip of paragraph 1, wherein the lumen of the dispensing tip includes a static mixer having one or more fixed baffles disposed therein.
3. The applicator tip of paragraph 1, wherein the at least one biologically active agent is a small molecule drug.
4. The applicator tip of paragraph 3, wherein the small molecule drug is selected from the group consisting of anesthetics, angiogenics, anti-spasmodics, non-steroidal anti-inflammatories, steroids, and combinations thereof.
5. The applicator tip of paragraph 1, wherein the at least one biologically active agent is a large molecule drug.
6. The applicator tip of paragraph 5, wherein the large molecule drug is a protein or a growth factor.
7. The applicator tip of paragraph 6, wherein the growth factor is TGF-β.
8. The applicator tip of paragraph 1, wherein the biologically active agent is coated on at least a portion of the mixing ball.
9. The applicator tip of paragraph 1, wherein the biologically active agent is entrapped in a component selected from the group consisting of liposomes and microspheres.
10. The applicator tip of paragraph 1, wherein the mixing ball is porous.
11. The applicator tip of paragraph 1, wherein the mixing ball is ionically charged.
12. A method of delivering a fluid and biologically active agent to tissue, the method comprising:
   introducing to a target tissue site a delivery device comprising:
      a fluid applicator including at least one fluid chamber containing a fluid and a fluid actuator for dispensing the fluid from the fluid chamber; and
      an applicator tip including a mixing chamber fluidly coupled to at least one fluid chamber of the fluid applicator, the mixing chamber containing at least one mixing ball and at least one biologically active agent and being fluidly coupled to a dispensing tip;
   activating the fluid actuator of the fluid applicator to urge fluid from the at least one fluid chamber of the fluid applicator into the mixing chamber of the applicator tip, such that movement of the fluid causes the mixing ball to move within the mixing chamber and mix the fluid with the biologically active agent disposed therein; and
   dispensing the fluid and biologically active agent through the dispensing tip to the target tissue site.
13. The method of paragraph 12, wherein the fluid applicator comprises two fluid chambers.
14. The method of paragraph 12, wherein the fluid actuator is selected from the group consisting of plungers, control buttons, and triggers.
15. The method of paragraph 12, wherein the fluid applicator further comprises an elongated sheath including at least one conduit fluidly connecting the at least one fluid chamber of the fluid applicator with the mixing chamber of the applicator tip.
16. The method of paragraph 12, wherein the dispensing tip includes at least one baffle fixedly secured therein.
17. The method of paragraph 12, wherein the biologically active agent comprises two or more biologically active agents.
18. The method of paragraph 17, wherein at least one of the biologically active agents is a dye.
19. The method of paragraph 18, further comprising waiting for the fluid to change color in the mixing chamber prior to dispensing the fluid and biologically active agent to the target tissue site.

## Claims

1. An applicator tip comprising:
a body portion including a proximal end having an inlet adapted for fluid communication with a fluid source, a distal end including an opening, and a mixing chamber disposed therebetween, the mixing chamber possessing at least one mixing ball and at least one biologically active agent disposed therein; and
a dispensing tip defining a lumen therethrough, the lumen being in fluid communication with the opening of the distal end of the body portion and including an outlet for passage of a fluid therethrough.

2. The applicator tip of claim 1, wherein the lumen of the dispensing tip includes a static mixer having one or more fixed baffles disposed therein.

3. The applicator tip of claim 1 or claim 2, wherein the at least one biologically active agent is a small molecule drug, preferably wherein the small molecule drug is selected from the group consisting of anesthetics, angiogenics, anti-spasmodics, non-steroidal anti-inflammatories, steroids, and combinations thereof.

4. The applicator tip of claim 1 or claim 2, wherein the at least one biologically active agent is a large molecule drug, preferably wherein the large molecule drug is a protein or a growth factor, more preferably wherein the growth factor is TGF-β.

5. The applicator tip of any preceding claim, wherein the biologically active agent is coated on at least a portion of the mixing ball.

6. The applicator tip of any preceding claim, wherein the biologically active agent is entrapped in a component selected from the group consisting of liposomes and microspheres.

7. The applicator tip of any preceding claim, wherein the mixing ball is porous.

8. The applicator tip of any preceding claim, wherein the mixing ball is ionically charged.

9. A method of delivering a fluid and biologically active agent to tissue, the method comprising:
introducing to a target tissue site a delivery device comprising:
a fluid applicator including at least one fluid chamber containing a fluid and a fluid actuator for dispensing the fluid from the fluid chamber; and
an applicator tip including a mixing chamber fluidly coupled to at least one fluid chamber of the fluid applicator, the mixing chamber containing at least one mixing ball and at least one biologically active agent and being fluidly coupled to a dispensing tip;
activating the fluid actuator of the fluid applicator to urge fluid from the at least one fluid chamber of the fluid applicator into the mixing chamber of the applicator tip, such that movement of the fluid causes the mixing ball to move within the mixing chamber and mix the fluid with the biologically active agent disposed therein; and
dispensing the fluid and biologically active agent through the dispensing tip to the target tissue site.
